# EUROPEAN PATENT APPLICATION

(11) **EP 2 755 153 A2**
(43) Date of publication of application: **16.07.2014**
(21) Application number: 13156345.4
(22) Date of filing: 22.02.2013
(51) Int. Cl.: G06F 19/00

(54) **Biological information management module, sleep monitor, and control apparatus**

(30) Priority: 11.01.2013 JP 2013003890
(71) Applicant: Tanita Corporation, Tokyo 174-8630 (JP)
(72) Inventor: Hotta, Junji, Tokyo, 174-8630 (JP)
(74) Representative: Grünecker, Kinkeldey, Stockmair & Schwanhäusser

(57) **Abstract**

A biological information management module (12) includes a first acquisition unit (14), a determination unit (18), and a generation unit (19). The first acquisition unit (14) acquires a plurality of pieces of biological information of different types for a living organism during sleep. The determination unit (18) determines the state of the living organism based on the pieces of biological information. The generation unit (19) generates an execution instruction when the determination unit (18) determines that the living organism is in a predetermined state. The execution instruction causes a first device (23) to execute a predetermined operation. The first device (23) executes the predetermined operation with respect to the living organism.

## Description

This application is based on an application No. 2013-003890 filed in Japan on January 11, 2013, the contents of which are hereby incorporated by reference.

### TECHNICAL FIELD

The present invention relates to biological information management modules, sleep monitors, and control apparatuses that cause a first device to perform a predetermined operation with respect to a living organism based on pieces of biological information acquired from the living organism.

### BACKGROUND ART

A wide variety of biological information, including blood pressure, an electrocardiogram, and brain waves, can be detected from a living organism, such as a human body. This biological information includes information that can vary significantly between a resting state and an irregular state, such as blood pressure. Acquiring and observing this biological information during predetermined states, such as the resting state and the irregular state, facilitates health management and allows for improved accuracy of diagnosis. A blood pressure measurement device has thus been proposed for measuring blood pressure after determining the state of a living organism by consecutively accumulating the pulse rate as biological information and comparing a newly measured pulse rate with the accumulated pulse rates (see Patent Literature 1).

### CITATION LIST

### Patent Literature

PTL 1: JP2005237472A

### SUMMARY OF INVENTION

The blood pressure measurement device in Patent Literature 1, however, requires detection of the pulse rate over an extended period of time in order to determine the state of the living organism. The subject therefore needs to wear the blood pressure measurement device for an extended period of time until a sufficient pulse rate sample is taken, which is uncomfortable for the subject. Furthermore, the blood pressure measurement device of Patent Literature 1 performs measurement when the pulse rate reaches the maximum value, the minimum value, and the average value measured in the past, yet these times may not correspond to the state of the living organism that desires to perform measurement. Moreover, for proper measurement of blood pressure, the subject needs to hold the position of blood pressure measurement, such as the arm, at a predetermined height with respect to the heart. Since an active subject cannot maintain such a measurement posture during activity, blood pressure cannot always be detected accurately.

The present invention has been conceived in light of the above problems, and it is an object thereof to provide a biological information management module, a sleep monitor, and a control apparatus that, without accumulating biological information, determine whether a living organism is in a predetermined state and drive a device based on the result of determination.

In order to solve the above problems, a biological information management module according to a first aspect comprises: a first acquisition unit configured to acquire a plurality of pieces of biological information of different types for a living organism during sleep; a determination unit configured to determine a state of the living organism based on the plurality of pieces of biological information during sleep; and a generation unit configured to generate, when the determination unit determines that the living organism is in a predetermined state, an execution instruction based on the state of the living organism determined by the determination unit, the execution instruction causing a first device to execute a predetermined operation with respect to the living organism.

In a biological information management module according to a second aspect, the generation unit preferably generates the execution instruction when the determination unit determines that the state of the living organism is at least one of an irregular state and a resting state.

A biological information management module according to a third aspect preferably further comprises a second acquisition unit configured to acquire first biological information measured by the first device based on the execution instruction generated during the irregular state and second biological information measured by the first device based on the execution instruction generated during the resting state, and the determination unit preferably determines presence of an abnormality in the living organism based on the first biological information and the second biological information.

In a biological information management module according to a fourth aspect, the plurality of pieces of biological information preferably include body motion of the living organism, and at least one of heart rate, pulse rate, and respiratory rate of the living organism.

In a biological information management module according to a fifth aspect, along with the execution instruction, the generation unit preferably generates a prohibiting instruction that, after execution of the predetermined operation in accordance with the execution instruction, prohibits execution of the predetermined operation for a predetermined period of time.

In a biological information management module according to a sixth aspect, the first acquisition unit preferably acquires a posture of the living organism as a portion of the plurality of pieces of biological information, and when the first acquisition unit acquires the posture of the living organism, the generation unit preferably includes the posture of the living organism in the execution instruction.

A biological information management module according to a seventh aspect preferably further comprises an output unit configured to output the execution instruction generated by the generation unit to the first device, the first device being detachable from the biological information management module.

A sleep monitor according to an eighth aspect comprises a biological information management module that includes a first acquisition unit configured to acquire a plurality of pieces of biological information of different types for a living organism during sleep; a determination unit configured to determine a state of the living organism based on the plurality of pieces of biological information during sleep; and a generation unit configured to generate, when the determination unit determines that the living organism is in a predetermined state, an execution instruction based on the state of the living organism determined by the determination unit, the execution instruction causing a first device to execute a predetermined operation with respect to the living organism.

A control apparatus according to a ninth aspect comprises a biological information management module that includes a first acquisition unit configured to acquire a plurality of pieces of biological information of different types for a living organism during sleep; a determination unit configured to determine a state of the living organism based on the plurality of pieces of biological information during sleep; and a generation unit configured to generate, when the determination unit determines that the living organism is in a predetermined state, an execution instruction based on the state of the living organism determined by the determination unit, the execution instruction causing a first device to execute a predetermined operation with respect to the living organism.

According to the present invention, it is possible, without accumulating biological information, to determine whether a living organism is in a predetermined state and to drive a device based on the result of determination.

### BRIEF DESCRIPTION OF DRAWINGS

The present invention will be further described below with reference to the accompanying drawings, wherein:
FIG. 1 is a functional block diagram schematically showing the internal structure of a sleep monitor that includes a biological information management module according to an embodiment of the present invention;
FIG. 2 is a graph showing changes over time in heart rate, respiratory rate, and body motion of a living organism during sleep as calculated by an analysis unit;
FIG. 3 is a flowchart showing first biological information management processing executed by the biological information management module;
FIG. 4 is a flowchart showing second biological information management processing executed by the biological information management module; and
FIG. 5 is a functional block diagram schematically showing the structure of a control apparatus incorporating the biological information management module.

### DESCRIPTION OF EMBODIMENTS

With reference to the drawings, the following describes an embodiment of the present invention.

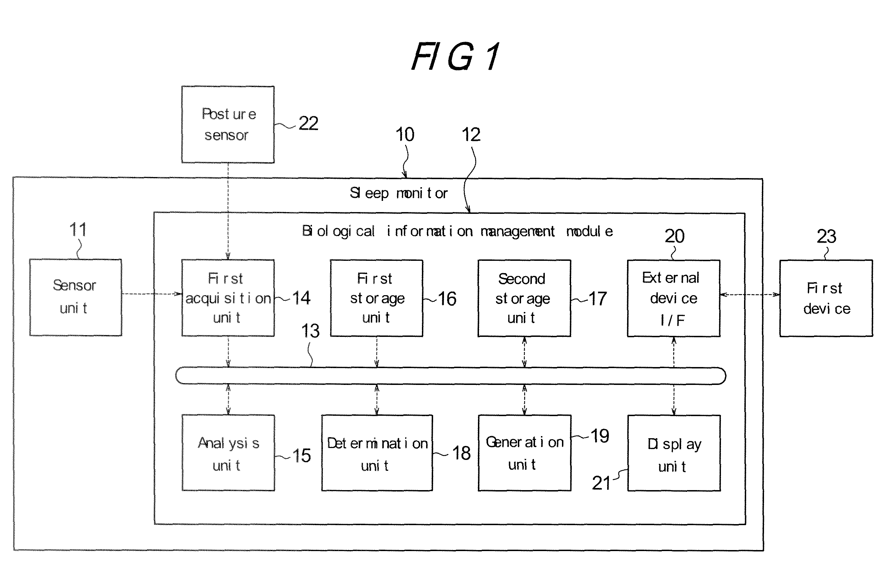

FIG. 1 is a functional block diagram schematically showing the internal structure of a sleep monitor that includes a biological information management module according to an embodiment of the present invention.

A sleep monitor 10 includes a sensor unit 11 and a biological information management module 12.

The sensor unit 11 includes a mat filled with a fluid, such as water or air, and a pressure sensor that detects pressure changes in the fluid. The sensor unit 11 detects vibrations of a subject, i.e. a living organism, sleeping on the mat. The vibrations detected by the sensor unit 11 include biological information during sleep such as heart rate (pulse rate), respiratory rate, and body motion of the living organism. The sensor unit 11 notifies the biological information management module 12 of the detected vibrations.

The biological information management module 12 includes a bus 13, a first acquisition unit 14, an analysis unit 15, a first storage unit 16, a second storage unit 17, a determination unit 18, a generation unit 19, an external device I/F 20 (output unit, second acquisition unit), and a display unit 21.

The bus 13 transmits information and commands between the first acquisition unit 14, the analysis unit 15, the first storage unit 16, the second storage unit 17, the determination unit 18, the generation unit 19, the external device I/F 20, and the display unit 21.

The first acquisition unit 14 acquires the vibrations detected by the sensor unit 11 as pieces of biological information during sleep that include biological information during sleep such as heart rate, respiratory rate, and body motion. The first acquisition unit 14 also detects posture information on the living organism from a posture sensor 22.

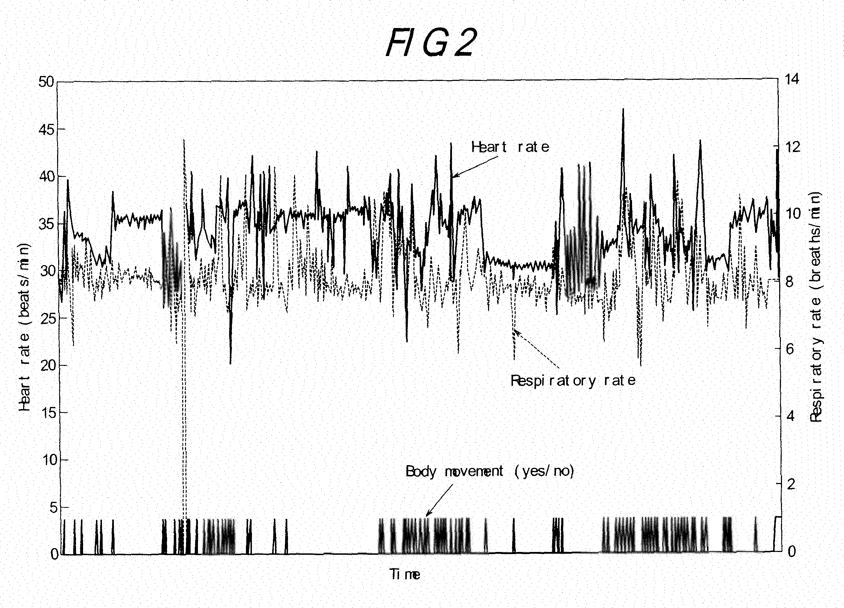

The analysis unit 15 performs a frequency analysis, for example, on the vibrations acquired by the first acquisition unit 14 and calculates the heart rate, respiratory rate, and body motion of the living organism, as shown in FIG. 2.

The first storage unit 16 is, for example, ROM and stores information to be determined in advance, such as a first threshold through a ninth threshold used by the determination unit 18.

The second storage unit 17 is, for example, RAM and stores information to be held temporarily, such as the heart rate, respiratory rate, and body motion of the living organism calculated by the analysis unit 15, first biological information and second biological information acquired from the external device I/F 20, as described below, and posture information acquired from the posture sensor 22, as also described below.

Based on the heart rate, respiratory rate, and body motion (biological information during sleep) of the living organism as calculated by the analysis unit 15, the determination unit 18 determines the state of the living organism. In order to determine the state, the determination unit 18 calculates the change in heart rate and respiratory rate during the most recent predetermined sample time. The calculated change is, for example, the variance, the standard deviation, the difference between the maximum value and the minimum value, or the like.

When no body motion is detected during the predetermined sample time, and the change in heart rate or pulse rate is no greater than the first threshold or the change in respiratory rate is no greater than a second threshold, the determination unit 18 determines that the living organism is in a resting state. When no body motion is detected during the predetermined sample time, and the change in heart rate or pulse rate is at least a third threshold or the change in respiratory rate is at least a fourth threshold, the determination unit 18 determines that the living organism is in a state of sudden change. When body motion is detected during the predetermined sample time, and the change in heart rate or pulse rate is at least a fifth threshold or the change in respiratory rate is at least a sixth threshold, the determination unit 18 determines that the living organism is in an irregular state. When body motion is detected during the predetermined sample time, and the change in heart rate or pulse rate is no greater than a seventh threshold or the change in respiratory rate is no greater than an eighth threshold, the determination unit 18 determines that the living organism is in a standard state.

During the above-described determination by the determination unit 18, the resting state refers to a state, such as during non-REM (Rapid Eye Movement) sleep, in which the living organism is calm both externally and mentally (in terms of brain activity). In this state, body motion is not present, and the heart rate or pulse rate and the respiratory rate are extremely stable. The state of sudden change refers to a state, such as REM sleep or so-called sleep paralysis, in which the living organism is externally calm yet mentally excited. While body motion is not present in this state, the heart rate or pulse rate and the respiratory rate exhibit relatively large change. The standard state is a typical active state for the living organism. In this state, body motion is present, and the heart rate or pulse rate and the respiratory rate exhibit change as compared to the resting state. The irregular state is thought to occur due to sleep apnea syndrome or when an abnormality occurs in the living organism. In this state, body motion is present, and the heart rate or pulse rate and the respiratory rate change greatly.

As described below, the determination unit 18 determines the presence of an abnormality in the living organism based on the first biological information and the second biological information acquired from a first device 23, which is a device external to the sleep monitor 10. The determination unit 18 compares the difference between the values corresponding to the first biological information and the second biological information with the ninth threshold. When the difference exceeds the ninth threshold, the determination unit 18 determines that an abnormality is present in the living organism.

When the determination unit 18 determines that the living organism is in a predetermined state, such as the resting state or the irregular state, the generation unit 19 generates an execution instruction, described below. Along with the execution instruction, the generation unit 19 also generates a prohibiting instruction, described below. The execution instruction and the prohibiting instruction include information that indicates the state. Furthermore, the execution instruction includes the latest posture information on the living organism.

The external device I/F 20 outputs the execution instruction and the prohibiting instruction generated by the generation unit 19 to the first device 23. In other words, the external device I/F 20 functions as the output unit. The external device I/F 20 also acquires the first biological information and the second biological information output by the first device 23, described below, and stores these pieces of information in the second storage unit 17. In other words, the external device I/F 20 also functions as the second acquisition unit.

The display unit 21 displays a variety of information detected by the sleep monitor 10. For example, when the determination unit 18 determines whether an abnormality is present in the living organism, the display unit 21 displays the result.

The posture sensor 22 is, for example, a camera having a posture detection function. The posture sensor 22 photographs an optical image of the living organism in synchronization with detection of vibrations by the sensor unit 11. The posture sensor 22 performs image analysis, such as contour extraction, on the photographed image of the living organism, detects the posture of the living organism, and notifies the first acquisition unit 14 of the result as posture information.

The first device 23 is a device external to the sleep monitor 10, as described above, and is attachable to the external device I/F 20 via a connector. When attached, the first device 23 can transmit a variety of information. The first device 23 is, for example, a blood pressure monitor that can perform predetermined operations with respect to the living organism for which the sensor unit 11 detects vibrations, such as measurement of blood pressure, recording of measurement results, calculation based on the measurement results, and transfer of information. Upon acquiring an execution instruction from the external device I/F 20, the first device 23 performs the above predetermined operations. As described above, the execution instruction includes posture information for the living organism, thus allowing the first device 23 to identify a posture of the living organism that could reduce the accuracy of measurement, such as a posture that compresses the first device 23. When the living organism is in such a posture, the first device 23 can associate an indication of low reliability of the measured blood pressure with the blood pressure value, as well as display and store such an indication.

The first device 23 recognizes the state of the living organism included in the execution instruction and associates the state of the living organism with the blood pressure value detected by performing the predetermined operations. For example, the first device 23 associates the irregular state with a blood pressure value detected based on an execution instruction occurring during the irregular state, handling the result as the first biological information. Furthermore, the first device 23 associates the resting state with a blood pressure value detected based on an execution instruction occurring during the resting state, handling the result as the second biological information. The first device 23 notifies the external device I/F 20 of the first biological information and the second biological information.

When the first device 23 acquires a prohibiting instruction from the external device I/F 20, the prohibiting instruction prohibits performance of the predetermined operations for a predetermined period of time after execution of the predetermined operations based on the execution instruction. During the predetermined period of time, the first device 23 is also, for example, prohibited by the prohibiting instruction from responding to an execution trigger caused by predetermined operations of a function of the first device 23 itself or a device external to the sleep monitor 10. As described above, the prohibiting instruction includes information that indicates the state of the living organism, and the predetermined period of time for prohibiting execution varies by state. For example, the predetermined period of time is three minutes during the irregular state, whereas the predetermined period of time is 45 minutes during the resting state.

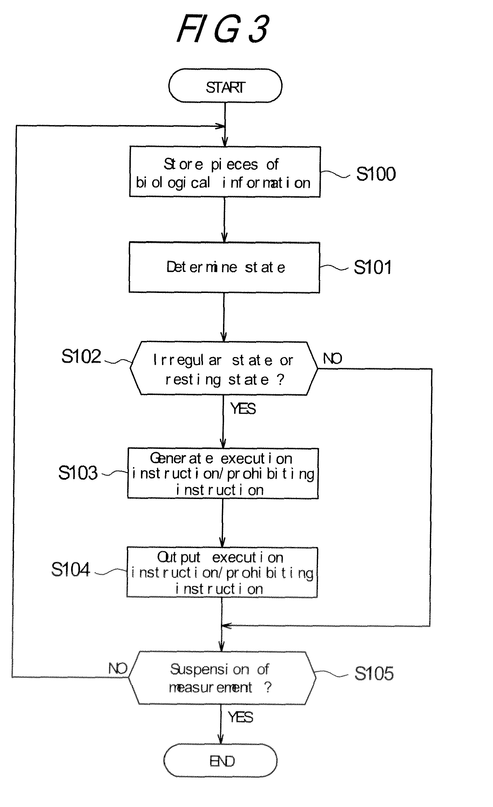

Next, first biological information management processing executed by the biological information management module 12 is described with reference to the flowchart in FIG. 3. The first biological information management processing starts upon the start of measurement of the pieces of biological information by the sleep monitor 10.

In step S100, the first acquisition unit 14 acquires the pieces of biological information from the sensor unit 11 and acquires the posture information from the posture sensor 22. The analysis unit 15 calculates the heart rate, respiratory rate, and body motion from the acquired pieces of biological information. Furthermore, the second storage unit 17 stores the calculated heart rate, respiratory rate, and body motion. Upon storage of the biological information, such as the heart rate, processing proceeds to step S101.

In step S101, the determination unit 18 reads the biological information for a predetermined time from the second storage unit 17 and calculates the change based on the read biological information. Furthermore, the determination unit 18 determines the state of the living organism based on the calculated change. Upon determination of the state of the living organism, processing proceeds to step S102.

In step S102, the determination unit 18 determines whether the state of the living organism determined in step S101 is at least one of the irregular state and the resting state. When the state of the living organism is one of these states, processing proceeds to step S103. When the state of the living organism is neither of these states, processing skips steps S103 and S104 and proceeds to step S105.

In step S103, the generation unit 19 generates an execution instruction and prohibiting instruction that include information on the state determined in step S101. Upon generation of the execution instruction and prohibiting instruction, processing proceeds to step S104.

In step S104, the external device I/F 20 notifies the first device 23 of the execution instruction and prohibiting instruction generated in step S103. After notification of the execution instruction and prohibiting instruction, processing proceeds to step S105.

In step S105, it is determined whether the input unit of the biological information management module 12 receives input indicating suspension of measurement by the sleep monitor 10. When no input indicating suspension of measurement is received, processing returns to step S100. When input indicating suspension of measurement is received, the first biological information management processing terminates.

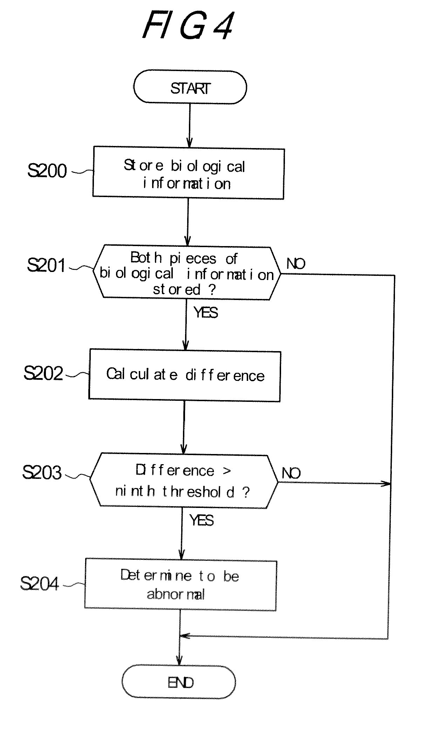

Next, second biological information management processing executed by the biological information management module 12 is described with reference to the flowchart in FIG. 4. The second biological information management processing starts when the external device I/F 20 acquires the first biological information or the second biological information from the first device 23.

In step S200, the second storage unit 17 stores the acquired first biological information or second biological information. Upon storage, processing proceeds to step S201.

In step S201, the determination unit 18 determines whether the second storage unit 17 currently stores both the first biological information and the second biological information. When both of these pieces of biological information are currently stored, processing proceeds to step S202. When at least one of these pieces of biological information is not currently stored, the second biological information management processing terminates.

In step S202, the determination unit 18 calculates the difference between the blood pressure values corresponding to the first biological information and the second biological information. Upon calculation of the difference, processing proceeds to step S203.

In step S203, the determination unit 18 determines whether the difference calculated in step S202 exceeds the ninth threshold. When the difference exceeds the ninth threshold, processing proceeds to step S204. When the difference is equal to or less than the ninth threshold, the second biological information management processing terminates.

In step S204, the display unit 21 displays that the living organism is in an abnormal state. After display, the second biological information management processing terminates.

With the above structure, the biological information management module of the present embodiment allows for determination of the state of the living organism by combining a plurality of pieces of biological information on the living organism during sleep. Accordingly, without accumulating biological information, the biological information management module of the present embodiment can determine the state of a living organism and drive a first device. Furthermore, by combining a plurality of pieces of biological information, the biological information management module of the present embodiment can determine the state of a living organism to a higher degree of accuracy than with a conventional approach, thereby allowing for execution of predetermined operations after a more accurate classification of the state of the living organism. By being used during sleep, the biological information management module of the present embodiment also frees the subject from the need to wear the first device 23 or the like for an extended period of time, thereby reducing discomfort experienced by the subject.

Since the first device 23 is detachable from the biological information management module 12, the biological information management module of the present embodiment allows for an external device to be selected according to the purpose of use and connected to the biological information management module 12 for use. In the present embodiment, one first device 23 is connected to the biological information management module 12, but a plurality of devices may be used.

By generating a prohibiting instruction, the biological information management module of the present embodiment can also prohibit the first device 23 from performing predetermined operations again for a predetermined period of time after execution of the predetermined operations. If, for example, the predetermined operations by the first device 23 are a strain on the body of the living organism, this prohibition can reduce the strain.

Furthermore, the biological information management module of the present embodiment can determine the presence of an abnormality in the living organism based on the blood pressure value in the irregular state and in the resting state. The determination of an irregular state by the determination unit 18 does not definitively establish the irregular state, but rather only indicates a state in which an abnormality may have occurred. Therefore, comparing the blood pressure values between the resting state and the irregular state allows for a determination of whether an abnormality has actually occurred in the living organism to a higher degree of accuracy than the mere determination of the irregular state.

The biological information management management module of the present embodiment can also include the posture information on the living organism detected by the posture sensor 22 in the execution instruction. As described above, the execution of predetermined processing may not be appropriate depending on the posture of the living organism. Since the first device 23 is notified of the posture information, the first device 23 can, for example, determine whether to execute the predetermined processing or to change the settings for the predetermined processing in accordance with the posture information.

Although the present invention has been described by way of an embodiment with reference to the accompanying drawings, it is to be noted that various changes and modifications will be apparent to those skilled in the art. Therefore, such changes and modifications are to be understood as included within the scope of the present invention.

For example, in the present embodiment, the first acquisition unit 14 acquires vibrations including the heart rate, respiratory rate, and body motion of a living organism as pieces of biological information, but the pieces of biological information may include other biological information. For example, a variety of information obtainable from a living organism may be used, such as pulse wave, weight, body temperature, an electrocardiogram, brain waves, optically detected blood sugar level, and gas within the living organism.

In the present embodiment, the first device 23 is a blood pressure monitor that measures blood pressure, but a device that measures different biological information may be used. For example, the device may measure a variety of information obtainable from a living organism, such as pulse rate, heart rate, respiratory rate, pulse wave, weight, body temperature, an electrocardiogram, brain waves, optically detected blood sugar level, and gas within the living organism. It is necessary, however, that the device measure biological information other than the biological information used as the above-described pieces of biological information. Furthermore, in the present embodiment, the first device 23 is a device that measures biological information (blood pressure), but instead of measuring biological information, the device may provide medical treatment or the like to the living organism. For example, when the irregular state is sleep apnea syndrome, the device can provide medical treatment to the living organism by supplying air to a CPAP worn by the subject.

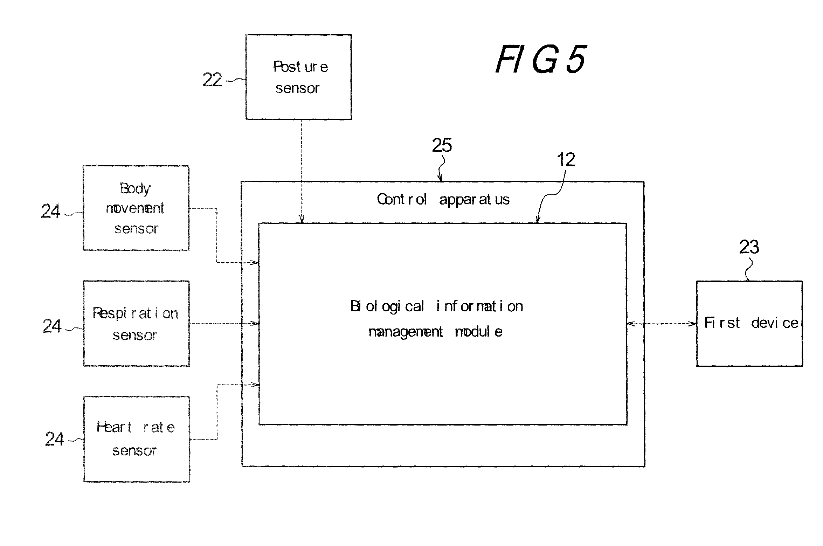

In the present embodiment, the biological information management module 12 is incorporated into the sleep monitor 10. As shown in FIG. 5, however, the biological information management module 12 may for example be structured as a control apparatus 25 that acquires the pieces of biological information from a plurality of sensors 24 and outputs the execution instruction to the first device 23.

In the present embodiment, the first device 23 is detachable from the biological information management module 12 but alternatively may form part of an integrated apparatus.

In the present embodiment, the determination unit 18 uses the changes in heart rate and respiratory rate to determine the state of the living organism, but alternatively the heart rate and respiratory rate may be used directly to determine the state of the living organism. For example, the state of the living organism may be determined by comparing the heart rate and the respiratory rate with a threshold. With this structure, the state of the living organism may be determined using the average values or moving average values of the biological information, and by using a variety of calculation methods, such as a cumulative method.

In the present embodiment, the posture sensor 22 detects posture based on a photograph image of the living organism, but a device that detects posture by another method may be used as the posture sensor 22. For example, posture may be detected using an inclination sensor, or by using a plurality of pressure detectors and observing the pressure distribution of the living organism.

### REFERENCE SIGNS LIST

| | |
|---|---|
| 10: | Sleep monitor |
| 11: | Sensor unit |
| 12: | Biological information management module |
| 13: | Bus |
| 14: | First acquisition unit |
| 15: | Analysis unit |
| 16: | First storage unit |
| 17: | Second storage unit |
| 18: | Determination unit |
| 19: | Generation unit |
| 20: | External device I/F |
| 21: | Display unit |
| 22: | Posture sensor |
| 23: | First device |
| 24: | Sensor |
| 25: | Control apparatus |

## Claims

1. A biological information management module comprising:
a first acquisition unit configured to acquire a plurality of pieces of biological information of different types for a living organism during sleep;
a determination unit configured to determine a state of the living organism based on the plurality of pieces of biological information during sleep; and
a generation unit configured to generate, when the determination unit determines that the living organism is in a predetermined state, an execution instruction based on the state of the living organism determined by the determination unit, the execution instruction causing a first device to execute a predetermined operation with respect to the living organism.

2. The biological information management module of claim 1, wherein the generation unit generates the execution instruction when the determination unit determines that the state of the living organism is at least one of an irregular state and a resting state.

3. The biological information management module of claim 2, further comprising:
a second acquisition unit configured to acquire first biological information measured by the first device based on the execution instruction generated during the irregular state and second biological information measured by the first device based on the execution instruction generated during the resting state, wherein
the determination unit determines presence of an abnormality in the living organism based on the first biological information and the second biological information.

4. The biological information management module of claim 1, wherein the plurality of pieces of biological information include body motion of the living organism, and at least one of heart rate, pulse rate, and respiratory rate of the living organism.

5. The biological information management module of claim 1, wherein along with the execution instruction, the generation unit generates a prohibiting instruction that, after execution of the predetermined operation in accordance with the execution instruction, prohibits execution of the predetermined operation for a predetermined period of time.

6. The biological information management module of claim 1, wherein the first acquisition unit acquires a posture of the living organism as a portion of the plurality of pieces of biological information, and
when the first acquisition unit acquires the posture of the living organism, the generation unit includes the posture of the living organism in the execution instruction.

7. The biological information management module of claim 1, further comprising:
an output unit configured to output the execution instruction generated by the generation unit to the first device, the first device being detachable from the biological information management module.

8. A sleep monitor comprising the biological information management module according to any one of claims 1-7.

9. A control apparatus comprising the biological information management module according to any one of claims 1-7.
